# EUROPÄISCHE PATENTSCHRIFT

(11) **EP 1 062 197 B1**
(45) Veröffentlichungstag und Bekanntmachung des Hinweises auf die Patenterteilung: **09.06.2004**
(21) Anmeldenummer: 99913227.7
(22) Anmeldetag: 04.03.1999
(51) Int. Cl.: C07C 51/50, C07C 51/44, C07C 57/04

(54) **REKTIFIKATION VON ACRYLSÄURE UNTER ZUSATZ EINES TENSIDS**
RECTIFICATION OF ACRYLIC ACID WITH THE ADDITION OF A TENSIDE
RECTIFICATION D'ACIDE ACRYLIQUE AVEC ADDITION D'UN TENSIOACTIF

(30) Priorität: 13.03.1998 DE 19810962
(43) Veröffentlichungstag der Anmeldung: 27.12.2000
(73) Patentinhaber: BASF AKTIENGESELLSCHAFT, 67056 Ludwigshafen (DE)
(72) Erfinder: HAMMON, Ulrich, D-68165 Mannheim (DE); HERBST, Holger, D-67227 Frankenthal (DE); NESTLER, Gerhard, D-67061 Ludwigshafen (DE)
(86) Internationale Anmeldenummer: PCT/EP1999/001414
(87) Internationale Veröffentlichungsnummer: WO 1999/047482

(56) Entgegenhaltungen:
- EP-A- 0 717 029
- WO-A-98/11048
- US-A- 4 600 795

## Beschreibung

Die vorliegende Erfindung betrifft ein neues Verfahren der rektifikativen Abtrennung von Acrylsäure aus einem Acrylsäure und eine höher als Acrylsäure siedende organische Flüssigkeit als Hauptbestandteile enthaltenden Gemisch

Acrylsäure, entweder für sich oder in Form ihrer Ester, ist insbesondere zur Herstellung von Polymerisaten für die verschiedensten Anwendungsgebiete, z.B. Verwendung als Klebstoffe, von Bedeutung.

Unter anderem ist Acrylsäure durch katalytische Gasphasenoxidation von Alkanen, Alkanolen, Alkenen oder Alkenalen erhältlich, die 3 C-Atome enthalten. Besonders vorteilhaft ist Acrylsäure z.B. durch katalytische Gasphasenoxidation von Propan oder Propen erhältlich. Als Ausgangsverbindungen sind aber auch solche denkbar, aus welchen sich die eigentliche C₃-Ausgangsverbindung während der Gasphasenoxidation erst intermediär bildet.

Dabei werden diese Ausgangsgase, in der Regel mit Inertgasen wie Stickstoff, CO, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z.B. Mo, V, W und/oder Fe enthaltende) Mischoxidkatalysatoren geleitet und oxidativ in die Acrylsäure umgewandelt (vgl. z.B. DE-A 4 405 059, EP-A 253 409, EP-A 92 097, DE-A 44 31 957, DE-A 44 31 949, CN-A 11 053 52 und WO 97/36849).

Aufgrund zahlreicher im Verlauf der katalytischen Gasphasenoxidation erfolgender Parallel- und Folgereaktionen sowie aufgrund der mitzuverwendenden inerten Verdünnungsgase wird bei der katalytischen Gasphasenoxidation jedoch keine reine Acrylsäure sondern ein Reaktionsgasgemisch erhalten, das im wesentlichen Acrylsäure, die inerten Verdünnungsgase und Nebenprodukte enthält, aus welchem die Acrylsäure abgetrennt werden muß. Neben von Acrylsäure vergleichsweise einfach zu entfernenden und bei Folgeverwendungen der Acrylsäure weniger störenden Nebenprodukten wie z.B. Essigsäure, enthält das Reaktionsgasgemisch häufig auch mit Acrylsäure eng verwandte und daher von Acrylsäure schwer abtrennbare niedere Aldehyde wie Formaldehyd, Acetaldehyd, Acrolein, Methacrolein, Propionaldehyd, n-Butyraldehyd, Benzaldehyd, Furfural und Crotonaldehyd sowie zusätzlich gegebenenfalls Maleinsäureanhydrid (bezogen auf die im Reaktionsgasgemisch enthaltene Menge an Acrylsäure beträgt die Gesamtmenge dieser, bei Folgeverwendungen häufig erheblich störenden, Nebenkomponenten in der Regel ≤ 2 Gew-%, meist ≥ 0,05 Gew.-%).

Die DE-A 44 36 243 betrifft ein Verfahren zur Abtrennung von Acrylsäure aus dem Reaktionsgasgemisch der katalytischen Gasphasenoxidation durch Gegenstromabsorption mit einer hochsiedenden inerten hydrophoben organischen Flüssigkeit, bei dem man das Reaktionsgasgemisch in einer Absorptionskolonne zu der absteigenden hochsiedenden inerten hydrophoben organischen Flüssigkeit im Gegenstrom führt, dem in der Absorptionskolonne in natürlicher Weise erfolgenden Absorptionsprozeß einen Rektifikationsprozeß überlagert, indem man der Absorptionskolonne eine über ihre, aufgrund ihres Kontaktes mit der Umgebungstemperatur erfolgende, natürliche Energieabgabe hinausgehende Energiemenge entzieht, und aus dem Acrylsäure und das Absorptionsmittel (Absorbens) als Hauptbestandteile sowie niedere Aldehyde und gegebenenfalls Maleinsäureanhydrid als Nebenbestandteile enthaltenden Flüssigkeitsablauf der Absorptionskolonne (Absorbat) die Acrylsäure rektifikativ abtrennt. Die dabei erhältliche Acrylsäure wird als Roh-Acrylsäure bezeichnet. Sie weist in der Regel eine Reinheit > 98 Gew.-% auf.

Als hochsiedende inerte hydrophobe organische Flüssigkeit (Absorbens) faßt die DE-A 44 36 243 dabei alle diejenigen Flüssigkeiten zusammen, deren Siedetemperatur bei Normaldruck (1 atm) oberhalb der Siedetemperatur der Acrylsäure liegt und die zu wenigstens 70 Gew.-% aus solchen Molekülen bestehen, die keine nach außen wirkende polare Gruppe enthalten und somit beispielsweise nicht in der Lage sind, Wasserstoffbrücken zu bilden. Dieser Begriffsinhalt gilt auch hier.

Aus der DE-PS 2 136 396 und der DE-A 43 08 087 ist ebenfalls bekannt, Acrylsäure aus dem Reaktionsgasgemisch der katalytischen Gasphasenoxidation von Propylen und/oder Acrolein durch Gegenstromabsorption mit einer hochsiedenden inerten hydrophoben organischen Flüssigkeit abzutrennen. Das Verfahren wird im wesentlichen so durchgeführt, daß man das Reaktionsgasgemisch in einer konventionellen Absorptionskolonne zu der absteigenden Absorptionsflüssigkeit im Gegenstrom führt, danach in einer Desorptionskolonne aus dem im wesentlichen aus Acrylsäure, dem Absorptionsmittel und Nebenkomponenten zusammengesetzten Flüssigkeitsablauf der Absorptionskolonne durch Strippen (Abstreifen) mit Inertgas die einfach abtrennbaren leichtflüchtigen Nebenkomponennten weitgehend entfernt und anschließend den Acrylsäure und das Absorbens als Hauptbestandteile enthaltenden Flüssigkeitsablauf der Desorptionskolonne zur Abtrennung von Roh-Acrylsäure rektifikativ behandelt.

Die DE-A 2 235 326 betrifft ebenfalls das Problem der rektifikativen Abtrennung von Acrylsäure aus deren Gemisch mit höher als Acrylsäure siedenden organischen Lösungsmitteln, wobei in diesem Stand der Technik als in Betracht zu ziehende organische Lösungsmittel vor allem auch höhere Alkohole oder Ester dieser oder anderer Alkohole, insbesondere mit Acrylsäure, genannt werden.

Nachteilig an der rektifikativen Abtrennung von Roh-Acrylsäure bzw. Acrylsäure aus Acrylsäure und eine hochsiedende organische Flüssigkeit als Hauptbestandteile enthaltenden Gemischen ist jedoch, daß sich im Verlauf der Rektifikation die Rektifikationsvorrichtungen (insbesondere die Verdampferoberfläche und die Kolonneneinbauten) mit Belag bedecken. Dies gilt insbesondere dann, wenn das rektifikativ aufzutrennende Gemisch niedere Aldehyde und gegebenenfalls Maleinsäureanhydrid als Nebenbestandteile enthält. Die vorgenannte Feststellung trifft selbst dann zu, wenn zu der rektifikativen Abtrennung Polymerisationsinhibitoren wie Phenothiazin, Paramethoxyphenol, Paranitrosophenol, Hydrochion, Hydrochinonmonomethylether Paraphenylendiamine, N-Oxylverbindungen und/oder Luft (wie sie z.B. in der DE-A 197 34 171 genannt werden) mitverwendet werden, um die Ausbildung polymerer (durch radikalische Polymerisation von Acrylsäure entstehende) Ablagerungen zu unterdrücken. Die Belagsbildung ist insofern von Nachteil, als sie von Zeit zu Zeit entfernt werden muß, was ein Abschalten des Rektifikationsbetriebes bedingt.

Die EP-A 717029 und die DE-A 2 235 326 empfehlen zur Minderung der Belagsbildung die Rektifikation unter Zusatz eines primären Amins und/oder dessen Salzen durchzuführen, doch Vermag diese Maßnahme nicht im vollen Umfang zu befriedigen.

Die EP-A 722926 empfiehlt als Probleinminderung das die rektifikativ abzutrennende Acrylsäure umfassende Einsatzgemisch der Rektifikationskolonne nicht unmittelbar zuzuführen, sondern zunächst in ein brüdenseitig mit dem Verstärkerteil der Rektifikationskolonne verbundenes beheiztes Verweilzeitgefäß zu leiten, in dem das Einsatzgemisch am Sieden gehalten wird und anstelle des Einsatzgemisches als solchem der Rektifikationskolonne die Sumpfflüssigkeit des Verweilzeitgefäßes zuzuführen, doch vermag auch diese Maßnahme das Problem nicht völlig zu lösen.

Aufgabe der vorliegenden Erfindung war daher ein neues Verfahren der rektifikativen Abtrennung von Acrylsäure aus einem Acrylsäure und eine höher als Acrylsäure siedende organische Flüssigkeit als Hauptbestandteile enthaltenden Gemisch, das bereits für sich alleine angewandt eine verminderte Belagsbildung und dadurch einen verlängerten Rektifikationsbetrieb ermöglicht, das aber insbesondere auch in Kombination mit den bereits bekannten Verfahren zur Minderung der Belagsbildung angewendet werden kann.

Demgemäß wurde ein Verfahren der rektifikativen Abtrennung von Acrylsäure aus einem Acrylsäure und eine höher als Acrylsäure siedende organische Flüssigkeit als Hauptbestandteile enthaltenden Gemisch gefunden, das dadurch gekennzeichnet ist, daß die Rektifikation unter Zusatz eines Tensids erfolgt.

Der Begriff Tensid meint dabei amphiphile Substanzen. Das sind solche Substanzen, die sowohl hydrophile als auch hydrophobe Gruppierungen aufweisen und Grenzflächenspannungen herabzusetzen vermögen. Hydrophile Gruppierungen sind solche, die in die wäßrige Phase hineingezogen werden, während hydrophobe Gruppierungen aus der wäßrigen Phase herausgedrängt werden.

Erfindungsgemäß geeignet sind vor allem solche Tenside, die beim Lösen in Wasser die Oberflächenspannung desselben herabzusetzen vermögen.

Während in Wasser lösliche Tenside in stark verdünnten wäßrigen Lösungen im wesentlichen als voneinander unabhängige Moleküle molekular gelöst vorliegen, wobei ihr amphiphiler Aufbau eine Anreicherung an der Wasseroberfläche als orientierte Adsorption bedingt, die eine Verringerung der Oberflächenspannung bewirkt, liegen wasserlösliche Tenside in konzentrierten wäßrigen Lösungen überwiegend micellar gelöst vor. D.h., die Tensidmoleküle ordnen sich in der wäßrigen Lösung überwiegend zu höheren Aggregaten, sogenannten Micellen an, in denen sie so orientiert sind, daß die hydrophilen Gruppierungen der wäßrigen Phase zugekehrt sind.und die hydrophoben Gruppierungen in das Innere der Micelle weisen.

Bei weiterer Steigerung der Tensidkonzentration nimmt im wesentlichen nur noch die Zahl der Micellen in der Volumeneinheit, nicht jedoch die der molekular gelösten Tensidmoleküle je Volumeneinheit zu.

Der Übergang von der wäßrigen molekularen Lösung zur wäßrigen micellaren Lösung erfolgt in Abhängigkeit von der Tensidkonzentration normalerweise relativ abrupt, was in einer entsprechend abrupten Änderung der Konzentrationsabhängigkeit zahlreicher makroskopischer Eigenschaften wie z.B. der Oberflächenspannung sichtbar wird und die kritische Micellbildungskonzentration (üblicherweise als molare Konzentration c.m.c. angegeben) definiert (Wendepunkt in der Konzentrationsabhängigkeit der Eigenschaft). Bei Konzentrationen oberhalb der kritischen Micellbildungskonzentration spricht man von micellaren Lösungen. Dabei soll der Begriff der "Lösung" zum Ausdruck bringen, daß das optische Erscheinungsbild einer micellaren wäßrigen Tensidlösung ebenso wie das einer molekularen wäßrigen Tensidlösung das einer klaren wäßrigen Lösung ist.

Typische Beispiele für erfindungsgemäß geeignete Tenside sind z.B. in Ullmanns Encyclopädie der technischen Chemie, Verlag Chemie, 4. Auflage, Bd. 22, S. 456 bis 515 angeführt.

Unter den Tensiden sind insbesondere jene erfindungsgemäß geeignet, die beim Lösen in Wasser bis zum Erreichen der kritischen Micellbildungskonzentration die Oberflächenspannung **σ** des reinen Wassers bei einer Temperatur von 20°C und einem Arbeitsdruck von 1 bar um wenigstens 15 %, bezogen auf den entsprechenden σ-Wert von reinem Wasser (73 mN/m), zu verringern vermögen.

D.h., erfindungsgemäß geeignet sind solche Tenside, bei denen die vorgenannte Erniedrigung des σ-Wertes von Wasser bis zum Erreichen der c.m.c. wenigstens 20%, oder wenigstens 25%, oder wenigstens 30%, oder wenigstens 35%, oder wenigstens 40%, oder wenigstens 50%, oder wenigstens 55%, oder wenigstens 60%, oder wenigstens 65%, oder wenigstens 70% beträgt.

Selbstverständlich eignen sich erfindungsgemäß aber auch solche Tenside, bei denen die vorgenannte Erniedrigung des σ-Wertes von Wasser bis zum Erreichen der c.m.c.wenigstens 75%, oder wenigstens 80%, oder wenigstens 90%, oder wenigstend 95% und mehr beträgt.

Prinzipiell können erfindungsgemäß sowohl anionische Tenside, nichtionische Tenside, kationische Tenside und/oder amphotere Tenside eingesetzt werden. Ihr HLB-Wert kann sowohl > 8 als auch ≤ 8 (z.B. 1 bis 8) betragen ( HLB ist die Abkürzung für "hydrophilic lipophilic balance"; der HLB-Wert kennzeichnet die Balance von hydrophilen und lipophilen(hydrophoben) Gruppen des Tensids; eine Definition des HLB-Werts ist gegeben in "Das Atlas HLB-System, Atlas Chemie GmbH, EC 10 G July 1971, und in "Classification of Surface Active Agents by HLB, W.C. Griffin, Journal of the Society of Cosmetic Chemists, 1 (1949), 311").

Bei erfindungsgemäßer Verwendung einer Tensidkombination ist lediglich darauf zu achten, daß die Einzelkomponenten untereinander verträglich sind. Dies ist beispielsweise nicht der Fall bei anionischen und kationischen Tensiden. Prinzipiell kann die Verträglichkeit in Vorversuchen in einfacher Weise überprüft werden.

Erfindungsgemäß bevorzugt werden nichtionische und/oder kationische Tenside verwendet.

Geeignete nichtionische Tenside sind beispielsweise alle Oxäthylate, die im allgemeinen durch Anlagerung von Ethylenoxid an Verbindungen mit beweglichen Protonen erhalten werden können.

Beispielhafte Vertreter solcher Oxäthylate sind die Oxäthylate von linearen und verzweigten, primären und sekundären Alkoholen mit 8 bis 30, vorzugsweise 12 bis 18 Kohlenstoffatomen, das sind insbesondere native und synthetische Fettalkohole (vgl. Ullmanns Encyclopädie der technischen Chemie, Verlag Chemie, 4. Auflage, Bd. 11,S. 427 ff.), die Oxäthylate von Alkylphenolen mit in den meisten Fällen verzweigten Octyl-, Nonyl- oder Dodecylresten, die Oxäthylate von Fettsäurealkanolamiden (z.b. Fettsäureethanolamiden) und Fettaminen (vgl. Ullmanns Encyclopädie der technischen Chemie, Verlag Chemie, 4. Auflage, Bd. 11, S. 447 ff) sowie die Oxäthylate von Estern der Fettsäuren mit Polyhydroxyverbindungen. Als teilweise mit Fettsäuren veresterte Polyhydroxyverbindungen kommen dabei z.B. Glycerin, Diglycerin, Polyglycerin, Erythrit, Pentaerythrit, Xylit, Sorbit, Mannit oder auch Saccharose und andere Glucoside in Betracht.

Der Oxäthylierungsgrad, das ist das Molverhältnis von addiertem Äthylenoxid pro Mol Substrat, kann in weiten Grenzen variieren. In der Regel beträgt er zwischen 3 und 40, häufig zwischen 6 und 30.

Selbstverständlich kann man auch mit Gemischen von Äthylenoxid und Propylenoxid oder mit Gemischen von Äthylenoxid und Butylenoxid oxalkylierte Substrate einsetzen, wobei die.Wahl des Mengenverhältnisses der beiden Epoxide variieren kann.

Als beispielhafte Vertreter der oben genannten nichtionischen Tenside seien Octylphenoloxäthylate mit einem Oxäthylierungsgrad von 4 bis 28, Fettalkoholoxäthylate von C₈- bis C₂₀- Fettalkoholen mit einem Oxäthylierungsgrad von 6 bis 28 und Oxäthylate von C₈bis C₁₈- Fettaminen mit einem Oxäthylierungsgrad von 6 bis 15 genannt.

Der Einbau von Oxypropyl-Gruppen in das Molekül eines Tensids erhöht dessen hydrophoben Charakter. Durch Anlagerung von Propylenoxid an ein niedermolekulares Startermolekül lassen sich so hydrophobe Grundkörper beliebiger Molmasse herstellen. Durch anschließende Oxäthylierung solcher erhaltener hydrophober Grundkörper gelangt man zu erfindungsgemäß geeigneten nichtionischen Tensiden, die man formal als Blockcopolymere des Propylenoxids und Äthylenoxids beschreiben kann. Bei Verwendung mehrfunktioneller Startermoleküle gehen von selbigem Verzweigungen aus. Beispielhaft hervorzuheben sind bezüglich einer erfindungsgemäßen Verwendung insbesondere die entsprechenden Derivate des Propylenglykols und des Äthylendiamins.

Durch Substitution des Wasserstoffatoms der endständigen Hydroxyl-Gruppe eines Oxalkylats durch hydrophobe Reste wie Benzyl-, Butyl- oder Methyl-Gruppen erhält man endständig blockierte Oxalkylate, insbesondere endständig blockierte Oxäthylate, die erfindungsgemäß gleichfalls einsetzbar sind.

Weiterhin kommen als erfindungsgemäß geeignete nichtionische Tenside Monomere, Oligomere und Polymere von Alkenylderivaten des 2,5-Pyrrolidindion und von Alkenylderivaten des Tetrahydrofuran-2,5-dion in Betracht. Beispielhaft genannt seien die Produkte TLA-1605 und TLA-627 der Fa. Texaco Additive Company, Belgien (vgl. GGC, Rev. 07/07/92, TB\TLA-1605.TB und GGC, 16/07/92, TB\TLA-627.TB). Das relative Molekulargewicht solcher Tenside kann z.B. 200 bis 5000 betragen. Insbesondere kommen auch die Tenside der US-A 3,271,296 als erfindungsgemäß zu verwendende Tenside in Betracht.

Im Unterschied zu nichtionischen Tensiden, die keine ionischen Gruppen aufweisen, handelt es sich bei den ionischen Tensiden um amphiphile Verbindungen, bei denen hydrophobe Reste als hydrophile Gruppen anionische bzw. kationische Gruppen tragen, denen die grenzflächenaktiven Eigenschaften der Substanz nur wenig beeinflussende Gegenionen gegenüberstehen.

Beispiele für erfindungsgemäß geeignete anionische Tenside sind zum Beispiel die Seifen, d.h., die Alkalimetall und Ammoniumsalze von natürlichen und synthetischen Fettsäuren (vgl. Ullmanns Encyclopädie der technischen Chemie, Verlag Chemie, 4. Auflage, Bd. 21, S. 209 ff). Zweckmäßige Vertreter der Seifen sind die Natrium-, Kalium- und Ammoniumseifen, insbesondere dann, wenn sie C₈- bis C₂₅-Seifen sind.

Weitere geeignete Vertreter anionischer Tenside sind die sogenannten Superseifen. Das sind die Alkalimetall- und Ammoniumsalze (insbesondere Na^{+,} K⁺ und NH₄⁺) von carboxymethylierten Oxäthylaten, wobei als Oxäthylate alle oben genannten in Betracht kommen.

Weitere erfindungsgemäß verwendbare anionische Tenside sind Sarkoside (Salze von Kondensationsprodukten aus Fett- und Aminosäuren) und Sulfonate. Sulfonate sind die Salze von Sulfonsäuren, bei denen eine Hydroxylsulfonyl-Gruppe über das Schwefelatom direkt an ein Kohlenstoffatom des hydrophoben Restes gebunden sind. Als kationische Gegenionen kommen vor allem das Natrium-, das Kalium-und das Ammoniumsalz sowie protonierte aliphatische Amine in Betracht .Beispielhafte Vertreter sind p-Alkylbenzolsulfonate mit 8 bis 20 Kohlenstoffatomen in der Alkylkette wie die Octyl- und Decyl- und Dodecylbenzolsulfonate. Insbesondere Alkylbenzolsulfonate mit 15 und mehr Kohlenstoffatomen im Alkylrest lösen sich gut in hydrophoben organischen Lösungsmitteln, wie sie auch in den erfindungsgemäß rektifikativ zu bearbeitenden Gemischen enthalten sein können. Alkylnaphthalinsulfonate wie Dipropyl- und Dibutylnaphthalinsulfonate und Alkansulfonate, insbesondere mit 12 bis 18 C-Atömen, eignen sich erfindungsgemäß ebenfalls als anionische Tenside. Schließlich seien als weitere Beispiele für erfindungsgemäß geeignete anionische Tenside die Alkali- und Ammoniumsalze (insbesondere Na⁺, K⁺ und NH4⁺) von α-Sulfofettsäureestern (z.B. von α-Sulfofettsäuremethylestern (auf Basis von Kokos-, Palmkern- oder Talgfettsäuren), von Sulfobernsteinsäuremono- und diestern (z.B. mit C₄- bis C₈-Alkanolen, Fettsäureethanolamiden oder deren Oxäthylaten (Oxäthylierungsgrad bevorzugt zwischen 3 und 12) als Veresterungsalkohol), von Alkoxy-, Acyloxy- und Acylaminoalkansulfonsäuren, von Halbestern der Schwefelsäure, der Phosphonsäure oder der Phosphorsäure mit primären oder sekundären C₈- bis C₂₅-Alkanolen (z.B. Hexadecanol oder Octadecanol) oder Äthoxylaten dieser Alkanole. Geeignet sind auch die Triethanolammoniumsalze der vorgenannten Verbindungen.

Tenside, deren hydrophiler Rest ein Kation ist, sind Kationen-Tenside. Die kationische Struktur kann im Molekül des Tensids bereits vorgegeben sein, wie in quartären Ammonium- oder Phosphoniumsalzen, sie kann sich aber auch erst nach Zusatz zum Acrylsäure enthaltenden, rektifikativ aufzutrennenden Gemisch ausbilden, wie z.B. in oxäthylierten Fettaminen. Als Kationen-Tenside im strengen Sinne sollen hier allerdings nur diejenigen verstanden werden, die nicht erst durch Protonierung mit Acrylsäure kationisch werden. Die erfindungsgemäß wichtigsten kationischen Tenside sind die quartären Stickstoffverbindungen und zwar die Tetraalkylammoniumsalze, die N,N-Dialkylimidazoline sowie die N-Alkylpyridiniumsalze (z.B. C₆- bis C₂₀- Alkylgruppen).

Als amphotere erfindungsgemäß geeignete Tenside sind z.B. die Ampholyte und Betaine zu nennen. Ampholyte sind Verbindungen mit mindestens einem beweglichen Proton. Ihre bekanntesten Vertreter sind die Aminocarbonsäuren.

Vorzugsweise werden erfindungsgemäß keine Tenside zugesetzt, die protonierte Sulfonsäuregruppen aufweisen. Besonders bevorzugt werden keine Tenside zugesetzt die protonierte Säuregruppen aufweisen, deren Acidität größer als die von Acrylsäure ist.

Ferner sind erfindungsgemäß solche Tenside bevorzugt, die keine -NH₂ und keine -NHR Gruppe (R = organischer Rest) aufweisen.

Bevorzugt sind ganz generell solche Tenside, deren Siedepunkt bei 1 bar > 200°C beträgt. Weiterhin ist es günstig, wenn sich das erfindungsgemäß zuzusetztende Tensid im rektifikativ zu behandelnden Gemisch bei Temperaturen von bis zu 200°C nicht zersetzt. Anwendungstechnisch zweckmäßig ist es ferner, wenn das zuzusetzende Tensid keine Halogenatome chemisch gebunden enthält und ökologisch möglichst unbedenklich ist.

Die erfindungsgemäß zuzusetzende Tensidmenge beträgt, bezogen auf das Gewicht des rektifikativ zu behandelnden, Acrylsäure enthaltenden, Gemisch in der Regel 10 bis 5000 gew.-ppm.

Das erfindungsgemäße Verfahren ist insbesondere anwendbar bei Gemischen, die zu 5 bis 25 Gew.-% Acrylsäure und zu 75 bis 95 Gew.-% organisches Lösungsmittel enthalten (jeweils bezogen auf das Gewicht des Gemisches).

Selbstverständlich ist das erfindungsgemäße Verfahren mit den in der EP-A 717029 und EP-A 722926 beschriebenen Verfahren in Kombination (jeweils getrennt oder zusammen) anwendbar.

Als organische Lösungsmittel werden vorzugsweise solche verwendet, die mit Acrylsäure im wesentlichen nicht reagieren, d.h., die sich inert verhalten.

Ferner eignet sich das erfindungsgemäße Verfahren insbesondere im Fall von Acrylsäure, deren gasphasenoxidative Herstellung einstufig ausgehend von Acrolein oder zweistufig ausgehend von Propylen über Acrolein erfolgt. Dies gilt insbesondere dann, wenn für die katalytische Gasphasenoxidation des Propylens ein Multimetalloxidkatalysator der allgemeinen Formel II

Mo₁₂BiₐFe_{b}X¹ _{c}X² _{d}X³ ₑX⁴ _{g}Oₙ (II),

in der die Variablen nachfolgende Bedeutung aufweisen:
- X¹: Nickel und/oder Kobalt,
- X²: Thallium, ein Alkalimetall und/oder ein Erdalkalimetall,
- X³: Phosphorat, Arsen, Bor, Antimon, Zinn, Cer, Blei und/oder Wolfram,
- X⁴: Silicium, Aluminium, Titan und/oder Zirkonium,
- a: 0,5 bis 5,
- b: 0,01 bis 3,
- c: 3 bis 10,
- d: 0,02 bis 2,
- e: 0 bis 5,
- g: 0 bis 10 und
- n: eine ganze Zahl, die durch die Wertigkeit und Häufigkeit der von Sauerstoff verschiedenen Elemente bestimmt wird,
und für die katalytische Gasphasenoxidation des Acroleins ein Multimetalloxidkatalysator der allgemeinen Formel III

Mo₁₂VₐW_{b}Cu_{c}Ni_{d}X¹ ₑX² _{f}X³ _{g}X⁴ ₕX⁵ ᵢOₙ (III),

in der die Variablen nachfolgende Bedeutung aufweisen:
- X¹: ein oder mehrere Alkalimetalle,
- X²: ein oder mehrere Erdalkalimetalle,
- X³: Chrom, Mangan, Cer, und/oder Niob,
- X⁴: Antimon und/oder Wismut,
- X⁵: Silicium, Aluminium, Titan und/oder Zirkonium,
- a: 1 bis 6,
- b: 0,2 bis 4,
- c: 0,5 bis 6,
- d: 0,2 bis 6,
- e: 0 bis 2,
- f: 0 bis 3,
- g: 0 bis 5,
- h: 0 bis 40,
- i: 0 bis 40 und
- n: eine ganze Zahl, die durch die Wertigkeit und Häufigkeit der von Sauerstoff verschiedenen Elemente bestimmt wird,
verwendet wird. Die Reaktionsgase der ersten Oxidationsstufe werden üblicherweise ohne Zwischenreinigung der zweiten Oxidationsstufe zugeführt.

Die üblicherweise angewendeten Reaktionsbedingungen können z.B. der DE-A 44 31 957 sowie der DE-A 44 31 949 entnommen werden.

Das erfindungsgemäße Verfahren ist insbesondere dann günstig, wenn das rektifikativ zu behandelnde Gemisch ein solches ist, das Acrylsäure und eine höher als Acrylsäure siedende inerte hydrophobe organische Flüssigkeit als Hauptbestandteile sowie niedere Aldehyde als Nebenbestandteile (in der Regel ≤ 2 Gew.-%) enthält. Dies gilt insbesondere dann, wenn das Gemisch aus den Reaktionsgasgemischen der vorgenannten Gasphasenoxidationen als Flüssigkeitsablauf einer Gegenstromabsorption mit anschließender Desorption durch Abstreifen mit einem Inertgas gemäß der DE-PS 21 36 396 oder der DE-A 43 08 087 oder als Flüssigkeitsablauf einer Gegenstromabsorption mit überlagerter Rektifikation gemäß der DE-A 44 36 243 erhalten wurde.

Dabei kommen als hochsiedende inerte hydrophobe organische Absorptionsflüssigkeiten insbesondere alle diejenigen in Betracht, die in der DE-A 21 36 396 und in der DE-A 43 08 087 empfohlen werden. Dies sind im wesentlichen Flüssigkeiten, deren Siedepunkt bei Normaldruck oberhalb von 160°C liegt. Beispielhaft genannt seien Mittelölfraktionen aus der Paraffindestillation, Diphenylether, Diphenyl, oder Mischungen der vorgenannten Flüssigkeiten wie z.B. ein Gemisch aus 70 bis 75 Gew.-% Diphenylether und 25 bis 30 Gew.-% Diphenyl. Günstig ist die Verwendung eines Gemisches bestehend aus einer Mischung aus 70 bis 75 Gew.-% Diphenylether und 25 bis 30 Gew.-% Diphenyl. Besonders günstig ist die Verwendung eines Gemisches bestehend aus einer Mischung aus 70 bis 75 Gew.-% Diphenylether und 25 bis 30 Gew.-% Diphenyl, sowie, bezogen auf diese Mischung, 0,1 bis 25 Gew.-% o-Dimethylphthalat. Häufig wird die hochsiedende inerte hydrophobe organische Flüssigkeit in der Absorptionskolonne dabei in solchen Mengen eingesetzt, daß der Flüssigkeitsablauf 5 bis 25, meist 5 bis 15 Gew.-% Acrylsäure enthält.

Vorzugsweise wird die erfindungsgemäße rektifikative Abtrennung von Acrylsäure bei reduziertem Druck, zweckmäßigerweise einem Kopfdruck ≤ 100 mbar, in der Regel 10 bis 100 mbar, vorgenommen. In entsprechender Weise betragen die Sumpftemperaturen 100 bis 220°C. Sie kann jedoch auch bei Drücken bis zu 1 bar durchgeführt werden.

Mit Vorteil erfolgt die erfindungsgemäße rektifikative Abtrennung von Acrylsäure kontinuierlich, wobei die Acrylsäure über Kopf oder Seitenabzug der Rektifikationskolonne bezogen wird. Das erfindungsgemäß zuzusetzende Tensid kann in zweckmäßiger Weise oberhalb der Entnahmestelle der Acrylsäure der Rektifikationskolonne zugeführt werden. Bemerkenswerterweise bewirkt die erfindungsgemäße Verfahrensweise sowohl eine Minderung der Belagsbildung im Verstärkungsteil als auch im Abtriebsteil der Rektifikationskolonne.

Als Rektifikationskolonne kommen alle gängigen Typen in Betracht. D.h. die Rektifikationskolonne kann z.B. eine Glockenboden-, Füllkörper- oder Packungskolonne sein. Vorzugsweise wird eine Glockenbodenkolonne angewendet. Mit Vorteil befindet sich die Stelle der kontinuierlichen Zufuhr des Flüssigkeitsgemisches, aus welchem die Acrylsäure rektifikativ abzutrennen ist, von der untersten theoretischen Trennstufe aus betrachtet etwa am Ende des ersten Drittels der Strecke zwischen unterster und höchstgelegener theoretischer Trennstufe.

Die im Rahmen der erfindungsgemäßen rektifikativen Abtrennung anfallende Sumpfflüssigkeit kann bei einer kontinuierlichen Ausführungsweise kontinuierlich entnommen und z.B. unmittelbar als Absorptionsflüssigkeit in der vorgeschalteten Absorptionsstufe wiederverwendet werden. Um die Betriebszeiten der Anlage zu erhöhen ist es dabei gegebenenfalls empfehlenswert, einen Teilstrom dieser hochsiedenden organischen Flüssigkeit abzuzweigen und erst nach Abtrennung des Absorptionsmittels in einer Aufarbeitungsstufe rückzuführen. Noch besser wird die gesamte Sumpfflüssigkeit vor ihrer Rückführung in die Absorptionskolonne durch eine solche Aufarbeitungsstufe geführt. Selbstverständlich kann das erfindungsgemäß zuzusetzende Tensid bereits in der die Abtrennung der Acrylsäure aus dem Reaktionsgasgemisch der katalytischen Gasphasenoxidation bewirkenden Absorptionskolonne oder in der gegebenenfalls nachgeschalteten Desorptionskolonne oder in den Zulauf zur Rektifikationskolonne zugesetzt werden, so daß eine direkte Zugabe in die Rektifikationskolonne teilweise oder vollständig entfällt. Selbstverständlich erfolgt das erfindungsgemäße Verfahren vorzugsweise im Beisein üblicher Mengen an üblichen Polymerisationsinhibitoren, vorzugsweise Phenothiazin. Normalerweise werden sie auf die Acrylsäuremenge (Gewicht) bezogen in Mengen von 50 bis 1000 ppm eingesetzt. Ferner wird die erfindungsgemäße Rektifikation vorzugsweise luftdurchströmt betrieben.

### Beispiele

Die nachfolgenden Beispiele wurden mit einem acrylsäurehaltigen Gemisch durchgeführt, das wie im Beispiel der EP-A 717029 gewonnen wurde.

### Das Gemisch enthielt

| | |
|---|---|
| 14,2 | Gew.-% Acrylsäure, |
| 0,02 | Gew.-% Essigsäure, |
| 0,01 | Gew.-% Furfurole, |
| 0,2 | Gew.-% Benzaldehyd, |
| 0,5 | Gew.-% Maleinsäureanhydrid, |
| 0,1 | Gew.-% Wasser, |
| 0,1 | Gew.-% Phenothiazin und als Restmenge bis 100 Gew.-% |

ein organisches Lösungsmittel bestehend aus 57,4 Gew.-% Diphenylether, 20,7 Gew.-% Diphenyl und 21,9 Gew.-% o-Dimethylphthalat.

### Beispiel 1

Das acrylsäurehaltige Gemisch wurde mit einer Temperatur von 25°C in einer Menge von 3 kg/h zwischen dem fünften und sechsten Boden (vom Verdampfer aus betrachtet) in eine 20 Glockenböden umfassende, luftdurchströmte Rektifikationskolonne geleitet. Die Rektifikationskolonne wurde mit einer Sumpftemperatur von 160°C und einem Sumpfdruck von 130 mbar sowie einem Kopfdruck von 80 mbar betrieben. Zwischen dem fünfzehnten und sechzehnten Boden (vom Verdampfer aus betrachtet) wurde über Seitenabzug pro Stunde 1300 ml Acrylsäure flüssig in einer Reinheit von 99,7 Gew.-% kontinuierlich entnommen. Das am Kolonnenkopf ankommende Gemisch aus Acrylsäure und den leichter als Acrylsäure siedenden Komponenten wurde kondensiert (600 ml/h), mit Phenothiazin (0,02 g/l) als Polymerisationsinhibitor versetzt und bis auf eine Entnahmemenge von 50 ml/h oberhalb des obersten Glockenbodens wieder in die Rektifikationskolonne zurückgeführt (oberer Rücklauf). Unterhalb des Seitenabzugs der Acrylsäure wurden 950 ml/h der ausgeschleusten Acrylsäure wieder in die Kolonne zurückgeführt (unterer Rücklauf). Die kontinuierlich entnommene Menge an Sumpfflüssigkeit betrug 2580 g/h. Nach einer Betriebsdauer von 140 Stunden mußte der Betrieb der Rektifikationskolonne infolge von Belagbildung auf den Kolonnenböden im Abtriebsteil und im Verstärkerteil abgebrochen werden.

### Beispiele 2 bis 9

Es wurde wie in Beispiel 1 verfahren, in den oberen Rücklauf wurden jedoch jeweils, bezogen auf die Rücklaufmenge, 60 bis 120 gew.-ppm eines Tensids und dem Gemischzulauf zur Rektifikationskolonne, bezogen auf die Zulaufmenge, 50 bis 200 gew.-ppm desselben Tensids zugegeben. Nachfolgende Tabelle zeigt die dabei erzielten Laufzeiten in Abhängigkeit von der jeweils verwendeten Sorte und Menge an zugegebenem Tensid.

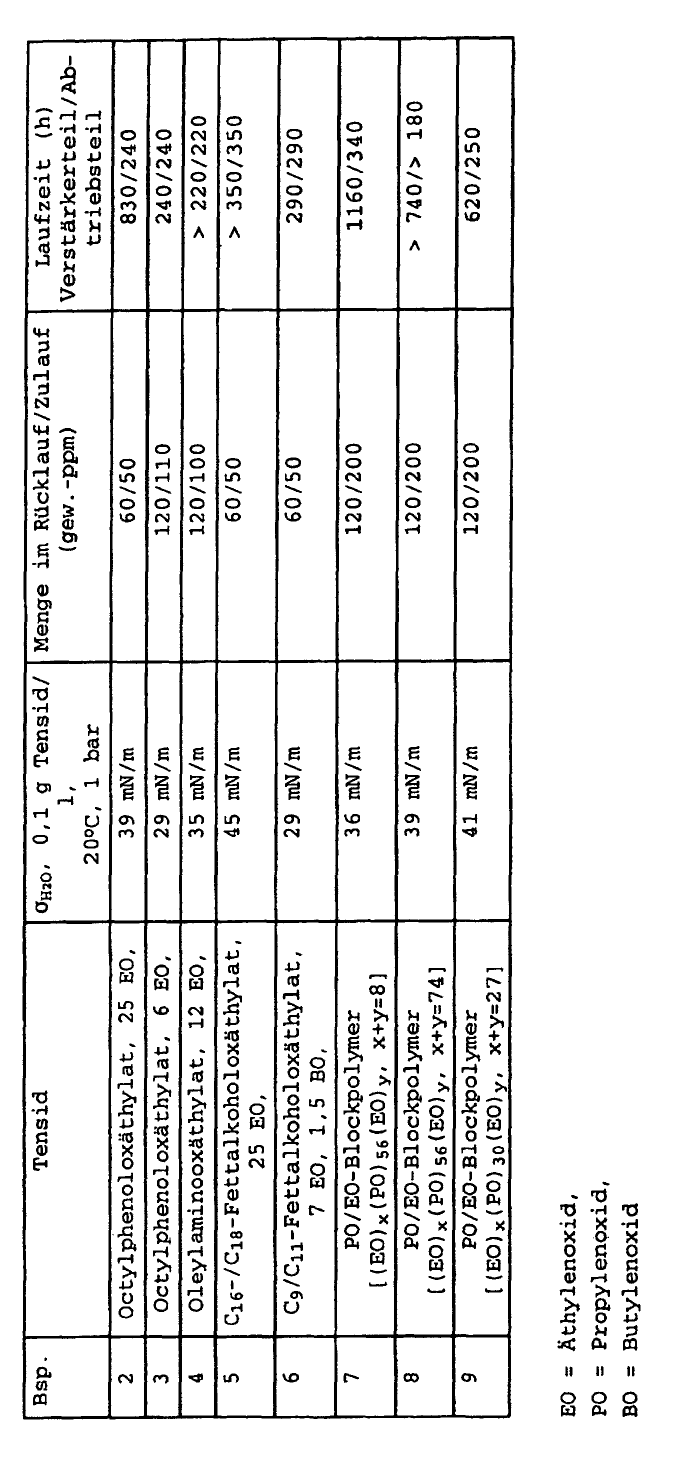

## Patentansprüche

1. Verfahren der rektifikativen Abtrennung von Acrylsäure aus einem Acrylsäure und eine höher als Acrylsäure siedende organische Flüssigkeit als Hauptbestandteile enthaltenden Gemisch, **dadurch gekennzeichnet, daß** die Rektifikation unter Zusatz eines Tensids erfolgt.

2. Verfahren nach Anspruch 1, **dadurch gekennzeichnet, daß** das zugesetzte Tensid ein nichtionisches Tensid ist.

3. Verfahren nach Anspruch 1 oder 2, **dadurch gekennzeichnet, daß** das zugesetzte Tensid so beschaffen ist, daß es beim Lösen in Wasser bis zum Erreichen der kritischen Micellbildungskonzentration die Oberflächenspannung des reinen Wassers bei einer Temperatur von 20°C und einem Arbeitsdruck von 1 bar, um wenigstens 15%, bezogen auf die entsprechende Oberflächenspannung des reinen Wassers, zu verringern vermag.

4. Verfahren nach einem der Ansprüche 1 bis 3, **dadurch gekennzeichnet, daß** die organische Flüssigkeit Diphenylether enthält.

5. Verfahren nach einem der Ansprüche 1 bis 4, **dadurch gekennzeichnet, daß** die höher als Acrylsäure siedende Flüssigkeit ein Gemisch bestehend aus einer Mischung aus 70 bis 75 Gew.-% Diphenylether und 25 bis 30 Gew.-% Diphenyl sowie, bezogen auf diese Mischung, 0,1 bis 25 Gew.-% o-Dimethylphthalat ist.

6. Verfahren nach einem der Ansprüche 1 bis 5, **dadurch gekennzeichnet, daß** das die Acrylsäure enthaltende Gemisch 5 bis 25 Gew.-% Acrylsäure enthält.

7. Verfahren nach einem der Ansprüche 1 bis 6, **dadurch gekennzeichnet, daß** die rektifikative Abtrennung bei einem Druck von 10 bis 100 mbar durchgeführt wird.

8. Verfahren nach einem der Ansprüche 1 bis 7, **dadurch gekennzeichnet, daß** die rektifikative Abtrennung im Beisein von Phenothiazin als Polymerisationsinhibitor durchgeführt wird.

9. Verfahren nach einem der Ansprüche 1 bis 8, **dadurch gekennzeichnet, daß** die abzutrennende Acrylsäure durch katalytische Gasphasenoxidation von C₃-Ausgangsverbindungen erzeugt worden ist.

10. Verfahren zur Herstellung von Acrylsäure durch katalytische Gasphasenoxidation einer C₃-Ausgangsverbindung, bei dem man das Reaktionsgasgemisch der Gasphasenoxidation in einer Absorptionskolonne zu einer absteigenden hochsiedenden inerten hydrophoben organischen Flüssigkeit im Gegenstrom führt, danach den Flüssigkeitsablauf der Absorptionskolonne in einer Desorptionskolonne mit Inertgas strippt und aus dem Flüssigkeitsablauf der Desorptionskolonne die Acrylsäure rektifikativ abtrennt, **dadurch gekennzeichnet, daß** die rektifikative Abtrennung unter Zusatz eines Tensids erfolgt.

## Claims

1. A process for the rectificative isolation of acrylic acid from a mixture containing, as main components, acrylic acid and an organic liquid having a higher boiling point than acrylic acid, wherein the rectification is carried out with the addition of a surfactant.

2. A process as claimed in claim 1, wherein the added surfactant is a nonionic surfactant.

3. A process as claimed in claim 1 or 2, wherein the added surfactant is such that, on dissolution in water up until reaching the critical micelle formation concentration, it is capable of reducing the surface tension of pure water at 20°C and at a working pressure of 1 bar by at least 15%, based on the corresponding surface tension of pure water.

4. A process as claimed in any of claims 1 to 3, wherein the organic liquid contains diphenyl ether.

5. A process as claimed in any of claims 1 to 4, wherein the liquid having a higher boiling point than acrylic acid is a mixture containing a mixture of from 70 to 75% by weight of diphenyl ether and from 25 to 30% by weight of diphenyl and, based on this mixture, from 0.1 to 25% by weight o-dimethyl phthalate.

6. A process as claimed in any of claims 1 to 5, wherein the mixture containing the acrylic acid contains from 5 to 25% by weight of acrylic acid.

7. A process as claimed in any of claims 1 to 6, wherein the rectificative isolation is carried out at from 10 to 100 mbar.

8. A process as claimed in any of claims 1 to 7, wherein the rectificative isolation is carried out in the presence of phenothiazine as a polymerization inhibitor.

9. A process as claimed in any of claims 1 to 8, wherein the acrylic acid to be isolated has been produced by catalytic gas-phase oxidation of C₃-starting compounds.

10. A process for the preparation of acrylic acid by catalytic gas-phase oxidation of a C₃-starting compound, in which the reaction gas mixture of the gas-phase oxidation is passed in countercurrent to a descending high-boiling inert hydrophobic organic liquid in an absorption column, the liquid discharge of the absorption column is then stripped with inert gas in a desorption column and the acrylic acid is isolated from the liquid discharge of the desorption column by rectification, wherein the rectificative isolation is carried out with the addition of a surfactant.

## Revendications

1. Procédé de séparation par rectification d'acide acrylique à partir d'un mélange contenant de l'acide acrylique et un liquide organique à point d'ébullition supérieur à celui de l'acide acrylique en tant que principaux constituants, **caractérisé en ce que** la rectification est entreprise avec addition d'un agent tensioactif.

2. Procédé selon la revendication 1, **caractérisé en ce que** l'agent tensioactif ajouté est un tensioactif non ionique.

3. Procédé selon la revendication 1 ou 2, **caractérisé en ce que** l'agent tensioactif permet, lors de sa dissolution dans l'eau jusqu'à atteindre la concentration critique de formation de micelles, de réduire la tension superficielle de l'eau pure à une température de 20°C et une pression de travail de 1 bar d'environ 15%, par rapport à la tension superficielle de l'eau pure.

4. Procédé selon l'une des revendications 1 à 3, **caractérisé en ce que** le liquide organique contient de l'éther diphénylique.

5. Procédé selon l'une des revendications 1à 4, **caractérisé en ce que** le liquide à point d'ébullition supérieur à celui de l'acide acrylique est un mélange constitué d'un mélange de 70 à 75% en poids d'éther diphénylique et de 25 à 30% en poids de diphényle ainsi que, par rapport à ce mélange, de 0,1 à 25% en poids de phtalate de o-diméthyle.

6. Procédé selon l'une des revendications 1 à 5, **caractérisé en ce que** le mélange contenant de l'acide acrylique contient de 5 à 25% en poids d'acide acrylique.

7. Procédé selon l'une des revendications 1 à 6, **caractérisé en ce que** la séparation par rectification est entreprise sous une pression de 10 à 100 mbar.

8. Procédé selon l'une des revendications 1 à 6, **caractérisé en ce que** la séparation par rectification est entreprise en présence de phénothiazine en tant qu'inhibiteur de polymérisation.

9. Procédé selon l'une des revendications 1 à 7, **caractérisé en ce que** l'acide acrylique à séparer a été obtenu par oxydation catalytique en phase gazeuse de composés de départ en C₃.

10. Procédé de préparation d'acide acrylique par oxydation catalytique en phase gazeuse d'un composé de départ en C₃, dans lequel on envoie le mélange réactionnel gazeux de l'oxydation en phase gazeuse dans une colonne d'absorption en contre-courant avec un liquide organique hydrophobe inerte à haut point d'ébullition, descendant, on envoie ensuite la sortie de liquide de la colonne d'absorption dans une colonne de désorption avec un gaz inerte et on sépare par rectification l'acide acrylique du liquide s'écoulant dans la colonne de désorption, **caractérisé en ce que** la séparation par rectification est entreprise avec addition d'un agent tensioactif.
